# DEMANDE DE BREVET EUROPEEN

(11) **EP 1 637 075 A1**
(43) Date de publication de la demande: **22.03.2006**
(21) Numéro de dépôt: 04370029.3
(22) Date de dépôt: 20.09.2004
(51) Int. Cl.: A61B 5/0452

(54) **Procédé et dispositif d'évaluation de la douleur chez un être vivant**

(71) Demandeur: Centre Hospitalier Regional Universitaire de Lille, 59037 Lille Cédex (FR); UNIVERSITE DU DROIT ET DE LA SANTE DE LILLE, 59800 Lille (FR)
(72) Inventeur: Logier, Régis, 59700 Marq en Baroeul (FR); Jeanne, Mathieu, 59000 Lille (FR); Tavernier, Benoît, 59130 Lambersart (FR)
(74) Mandataire: Matkowska, Franck

(57) **Abrégé**

Le procédé d'évaluation de la douleur ressentie par un être vivant, comprend les étapes principales suivantes :
- acquisition d'un signal cardiaque analogique de l'être vivant,
- échantillonnage de ce signal cardiaque et construction d'une série RR constituée d'une pluralité d'échantillons (RRᵢ) représentant les intervalles de temps qui séparent deux battements cardiaques successifs,
- sélection de (N) échantillons (RRi) dans une fenêtre temporelle principale de durée (n) prédéfinie,
- découpage de cette fenêtre principale en (m) sous-fenêtres (Fⱼ),
- calcul pour chaque sous-fenêtre (Fⱼ) d'un paramètre intermédiaire (Aⱼ) à partir des échantillons (RRi) compris dans la sous-fenêtre (Fⱼ),
- calcul d'un paramètre final qui est fonction des paramètres intermédiaires (Aⱼ).

## Description

### Domaine de l'invention

La présente invention concerne un procédé et un dispositif d'évaluation de la douleur chez un être vivant, basés sur l'analyse de la variabilité du rythme cardiaque. Elle trouve avantageusement son application à la mesure de la douleur ressentie par un être vivant conscient ou inconscient. Plus particulièrement, l'invention trouve son application dans le domaine chirurgical à la mesure du niveau de douleur ressentie par un patient conscient sous anesthésie locale du type péridurale, ou du niveau de douleur ressentie par un patient inconscient sous anesthésie générale.

### Art antérieur

Dans le domaine médical ou chirurgical, il est d'une manière générale important de pouvoir connaître le niveau de douleur ressentie par un patient, afin notamment de pouvoir prendre en compte et traiter de manière optimale cette douleur.

La méthode d'évaluation de la douleur la plus répandue à ce jour est une méthode subjective, basée sur l'utilisation d'une réglette permettant au patient d'indiquer au moyen d'un curseur ou équivalent le niveau de douleur qu'il ressent sur une échelle de douleur pré-établie. Cette méthode présente l'inconvénient d'une part d'être purement subjective, et n'est donc pas réellement fiable, et d'autre part ne peut être envisagée qu'avec des patients conscients.

Au surplus, dans le cas d'un patient conscient sous anesthésie locale, tel que par exemple un patient sous péridurale, la méthode d'évaluation subjective précitée ne peut être utilisée pour contrôler automatiquement dans le temps l'administration des produits anesthésiques.

Dans le domaine de l'anesthésie générale, il est également intéressant de pouvoir connaître le niveau de douleur ressentie par le patient inconscient. La connaissance de ce niveau de douleur permet d'adapter au mieux l'administration des produits analgésiques.

Pour les raisons ci-dessus, il y a un intérêt important à trouver une méthode objective d'évaluation de la douleur ressentie par un être vivant, ladite méthode devant se traduire par un calcul automatique d'un paramètre mesurant le niveau de douleur.

### Objectif de l'invention

Un premier objectif de l'invention est de proposer une méthode d'évaluation objective du niveau de douleur ressentie par un être vivant.

Un deuxième objectif de l'invention est plus particulièrement de proposer une méthode d'évaluation objective du niveau de douleur ressentie par un être vivant conscient.

Un troisième objectif de l'invention est plus particulièrement de proposer une méthode d'évaluation objective du niveau de douleur ressentie par un être vivant conscient et non ventilé mécaniquement (c'est-à-dire un être vivant dont la fréquence respiratoire est quelconque et variable, et n'est pas imposée par un dispositif de ventilation contrôlée par contraste notamment avec un patient sous anesthésie générale).

Un quatrième objectif de l'invention est de proposer une méthode d'évaluation objective du niveau de douleur ressentie par un être vivant, ladite méthode pouvant s'appliquer aussi bien à un être vivant conscient ou à un être vivant inconscient, et en particulier à un être vivant sous anesthésie générale.

### Résumé de l'invention

Au moins le premier objectif précité est atteint au moyen du procédé de la revendication 1, lequel procédé permet, à partir d'un signal cardiaque de l'être vivant, de calculer automatiquement au moins un paramètre objectif quantifiant le niveau de douleur ressenti par l'être vivant.

Dans le présent texte, et notamment dans les revendications, on désigne par les termes « signal cardiaque », tout signal physique caractéristique du rythme (ou de la fréquence) cardiaque instantanée de l'être vivant. Pour la mise en oeuvre de l'invention, différentes techniques invasives ou non invasives peuvent être utilisées pour acquérir ce signal cardiaque. Une technique invasive connue consiste par exemple à utiliser un capteur de pression sanglante relié à un cathéter introduit dans une artère. Parmi les méthodes non invasives connues (et pour lesquelles on optera de préférence), on peut citer par exemple l'utilisation d'un capteur de pouls infrarouge, l'utilisation d'un capteur à ultrasons permettant la détection des cycles cardiaques, du type du capteur mis en oeuvre dans un cardiotocographe, ou encore l'acquisition d'un signal électrocardiographique (ECG). L'acquisition d'un signal électrocardiographique (ECG) est en pratique la méthode la plus couramment utilisée, car outre son caractère non invasif, elle permet d'obtenir un signal cardiaque plus précis que celui obtenu par exemple au moyen d'un capteur de pouls infrarouge.

Dans le présent texte, et notamment dans les revendications, on désigne d'une manière générale par les termes « série RR », une série de plusieurs échantillons successifs (RRᵢ), obtenus après échantillonnage d'un signal cardiaque analogique caractéristique du rythme cardiaque de l'être vivant, chaque échantillon (RRi) caractérisant d'une manière générale un intervalle de temps entre deux battements cardiaques successifs.

Dans l'exemple préféré de réalisation décrit ci-après en référence aux figures annexée, cette série RR est plus particulièrement construite à partir des ondes R d'un signal ECG. Ceci n'est toutefois pas limitatif de l'invention. Dans le cas d'un signal cardiaque type ECG, on peut construire la série dite « RR » en utilisant les autres ondes de dépolarisation (P, Q, S ou T) du signal ECG pour construire la série RR, la précision étant toutefois moins bonne qu'en utilisant les ondes R du signal ECG. Egalement, lorsque le signal cardiaque n'est pas un signal ECG, les échantillons de la série RR ne sont pas calculés en déterminant l'intervalle de temps séparant deux ondes R successives du signal ECG, mais sont d'une manière plus générale déterminés en détectant dans le signal cardiaque l'intervalle de temps entre deux battements cardiaques successifs.

### Description des figures

D'autre caractéristiques et avantages de l'invention apparaîtront plus clairement à la lecture de la description détaillée ci-après d'un exemple préféré de réalisation du procédé de l'invention, laquelle description détaillée est donnée à titre d'exemple non limitatif et non exhaustif, et en référence aux dessins annexés sur lesquels :
- la figure 1 représente de manière schématique les principaux éléments d'un exemple de système d'acquisition et de traitement d'un signal ECG utilisé pour mettre en oeuvre le procédé de l'invention,
- la figure 2 représente l'ensemble d'ondes (PQRST) caractéristique d'un battement cardiaque dans un signal ECG,
- la figure 3 représente un exemple de signal numérique ECG, obtenu après échantillonnage d'un signal ECG analogique,
- la figure 4 représente un exemple de série RR (encore désigné signal RR ) construite à partir du signal de la figure 3, et
- la figure 5 représente un exemple de série RR après filtrage et normalisation.

### Description détaillée

### Système d'acquisition et de traitement du signal cardiaque

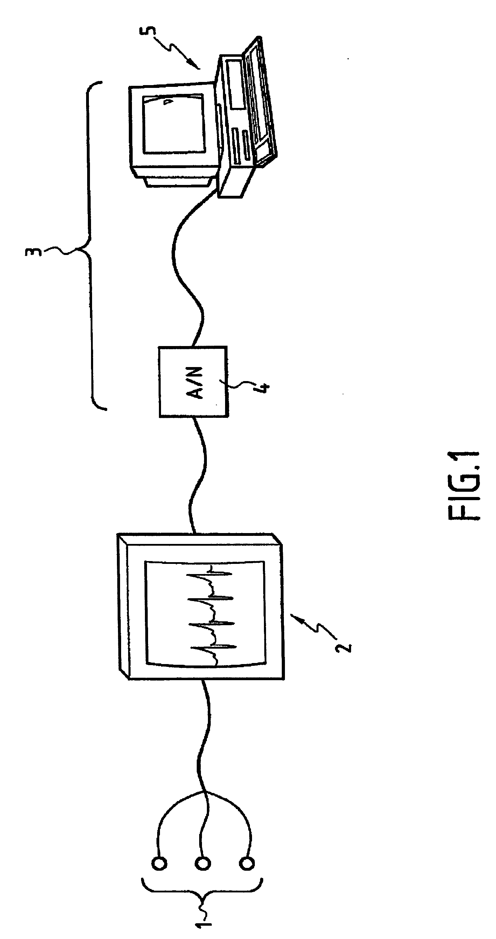

On a représenté sur la figure 1 un exemple de système d'acquisition et de traitement du signal cardiaque d'un être vivant (désigné par la suite « patient ») qui est utilisé pour la mise en oeuvre du procédé de l'invention.
Ce système comporte :
- des moyens usuels d'acquisition d'un signal ECG, comprenant plusieurs électrodes de mesure 1 reliées en entrée à un moniteur électrocardiographique (ECG) 2,
- des moyens 3 de traitement du signal ECG délivré en sortie par le moniteur ECG 2.

Les moyens de traitement 3 du signal ECG comprennent un convertisseur analogique/numérique 4, et une unité de traitement programmée 5. L'entrée du convertisseur 4 est reliée à la sortie du moniteur ECG2, et la sortie du convertisseur 4 est reliée à un port d'entrée de l'unité de traitement 5. Dans un exemple particulier de réalisation, non limitatif de l'invention, l'unité de traitement 5 est constituée par un micro-ordinateur, le convertisseur 4 étant relié à un port série RS232 de ce micro-ordinateur.

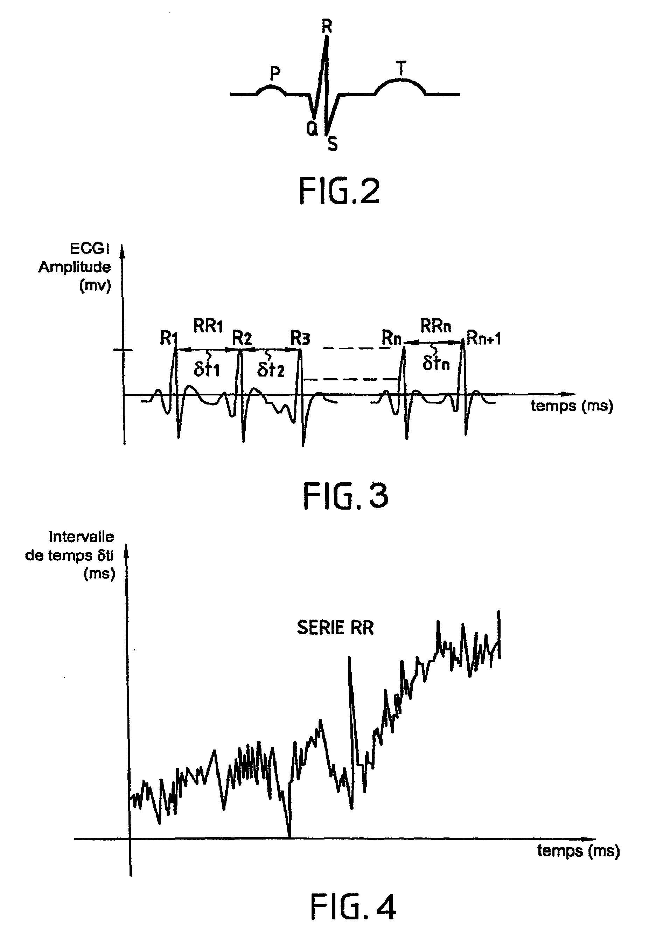

En fonctionnement, les électrodes 1 sont appliquées sur le corps d'un patient, et le moniteur ECG délivre en sortie de manière usuelle un signal électrique analogique, dit signal ECG, qui pour chaque battement cardiaque, a la forme du signal représenté a la figure 2.

En référence à la figure 2, pour chaque battement cardiaque, ce signal électrocardiographique (ECG) est constitué d'un ensemble d'ondes électriques :
- l'onde P, qui correspond à la dépolarisation des oreillettes, et qui présente une faible amplitude et une forme de dôme ;
- l'espace PQ qui traduit le temps de conduction auriculo-ventriculaire ;
- l'onde R considérée en pratique comme marqueur de la systole ventriculaire, ou du battement cardiaque, le complexe QRS reflétant la contraction ventriculaire, et
- l'onde T qui reflète la repolarisation ventriculaire.

Ce signal ECG analogique est numérisé par le convertisseur 4, avec une fréquence d'échantillonnage (fc) prédéterminée, valant par exemple 256 Hz.

Le signal échantillonné délivré en sortie du convertisseur 4, (signal représenté sur la figure 3) est traité par l'unité de traitement 5, au moyen d'un logiciel de traitement spécifique (logiciel d'évaluation de la douleur) qui est décrit en détail ultérieurement. Ce logiciel d'évaluation de la douleur est stocké en mémoire de l'unité de traitement 5, et permet, lorsqu'il est exécuté, de calculer automatiquement, à partir du signal numérique délivré par le convertisseur analogique/numérique 4, deux paramètres distincts (AUCmax et AUCmoy) mesurant le niveau de douleur du patient.

Une variante préférée de ce logiciel d'évaluation de la douleur va à présent être détaillée.

### Exemple d'algorithme du logiciel d'évaluation de la douleur

Dans une variante préférée de réalisation de l'invention, les principales étapes successives de l'algorithme du logiciel d'évaluation de la douleur sont les suivantes :
1. Acquisition des échantillons RRi et ré-échantillonnage à une fréquence f prédéfinie
2. Sélection des échantillons RRi compris dans une fenêtre temporelle principale de n secondes (n>1/f)
3. Filtrage
4. Normalisation du signal
5. Détection des minimums dans la fenêtre principale
6. Découpage de la fenêtre temporelle principale en m [m ≥2] sous-fenêtres (Fⱼ) et calcul pour chaque sous-fenêtre (Fⱼ) de l'aire (Aⱼ) délimitée par l'enveloppe inférieure (Cⱼ) reliant les minimums détectés à l'étape précédente
7. Calcul des paramètres : AUCmoy et AUCmax
8. Décalage, d'un pas temporel valant p secondes (p<n), de la fenêtre temporelle de n secondes, et réitération du calcul à partir de l'étape 2.

En pratique, le système peut être programmé pour être utilisé en temps réel ou en temps différé.

Lorsque le système est utilisé en temps différé, on réalise dans un premier temps l'étape 1 en temps réel en sorte d'acquérir tous les échantillons RRi sur toute la période d'analyse souhaitée ; l'intégralité de ces échantillons RRi successifs est stockée dans un fichier d'acquisition en mémoire de l'unité de traitement l'étape 1. Dans un second temps, les étapes 2 à 8 sont effectuées en boucle, en différé, sur les valeurs d'intervalle RRi stockée dans le fichier d'acquisition.

Lorsque le système fonctionne en temps réel, l'étape d'acquisition 1 d'une part, et les autres étapes de traitement 2 à 8 d'autre part sont exécutées par deux modules logiciels distincts fonctionnant en parallèle, le premier module d'acquisition (étape 1) alimentant le second module de traitement et de calcul (étapes 2 à 8) par l'intermédiaire d'un fichier tampon ou équivalent.

Les étapes 1 à 8 vont a présent être détaillées.

### Etape 1 : Acquisition des échantillons RRi et ré-échantillonnage à une fréquence f prédéfinie

L'acquisition des échantillons RRi est réalisée par un premier sous-module logiciel qui est alimenté en entrée avec les données numériques successives constitutives du signal ECG numérisé (signal de la figure 3) délivré par le convertisseur analogique numérique 4. Chaque donnée (ou point) du signal ECG est définie par l'amplitude instantanée ECGᵢ du signal ECG, et par l'instant tᵢ d'échantillonnage (tᵢ = nᵢ /fc, avec nᵢ numéro d'échantillon et fc représentant la fréquence d'échantillonnage du convertisseur 4).

Le premier sous-module d'acquisition des échantillons RRᵢ est conçu pour détecter automatiquement chaque pic Ri successif dans le signal numérique délivré par le convertisseur 4, et pour construire automatiquement une série RR (figure 4) constituée d'une succession de d'échantillons RRᵢ. Chaque échantillon RRᵢ est défini par le couple de coordonnées : ti [un instant (ou numéro) d'échantillonnage] ; intervalle de temps δti (exprimé en multiple de la fréquence d'échantillonnage fc) séparant un pic Rᵢ du pic suivant Rᵢ₊₁(dans une autre variante il pourrait s'agir du pic précédent Rᵢ₋₁).

De manière usuelle en soi, l'onde R étant le plus souvent la partie la plus fine et la plus ample du QRS, elle est de préférence utilisée pour détecter le battement cardiaque avec une très bonne précision, l'intervalle de temps δti correspondant en pratique au temps séparant deux battements cardiaques successifs. Néanmoins, dans une autre variante, on pourrait envisager d'utiliser d'autres ondes (par exemple onde Q ou onde S) d'un battement cardiaque du signal ECG pour détecter et construire la série RR.

La série RR (figure 4) délivrée par le premier sous-module précité est ré-échantillonnée automatiquement par un second sous-module logiciel à une fréquence prédéfinie f, qui est de préférence inférieure à la fréquence d'échantillonnage fc (par exemple, pour une fréquence d'échantillonnage fc valant 256 Hz, on fixera la fréquence f de ré-échantillonnage à 8hz). L'objectif de ce ré-échantillonnage est d'obtenir en sortie une série RR dont les échantillons RRᵢ sont équidistants d'un point de vue temporel, c'est-à-dire en d'autres termes une série RR dont les instants d'échantillonnages sont réguliers. Ce ré-échantillonnage est réalisé de manière connue en soi par interpolation, et par exemple par interpolation linéaire.

### Etape 2: Sélection des échantillons RRi compris dans une fenêtre temporelle principale de n secondes (n>1/f)

Cette étape revient à isoler un nombre N d'échantillons RRi successifs (N=n.f). A titre indicatif, on choisit par exemple une fenêtre principale de 64 secondes (n=64), ce qui correspond à 512 échantillons RRi successifs (N=512), pour une fréquence f de ré-échantillonnage de 8hz.

Les étapes suivantes 3 à 7 sont appliquées aux échantillons compris dans cette fenêtre principale.

### Etape 3 : filtrage / [f1 ; f2]

Cette étape consiste à appliquer un filtre passe-bande sur les échantillons de la série RR compris dans la fenêtre principale en sorte de conserver uniquement les fréquences comprises dans une bande de fréquence prédéfinie [f1 ; f2].

Plus particulièrement, la bande de fréquence [f1 ; f2] est égale ou est comprise dans la bande de fréquences [0.1 Hz ; 5Hz]. De préférence, la bande de fréquence [f1 ; f2] est égale à [0,125Hz ; 1 Hz].

Pour réaliser cette étape de filtrage passe-bande, on utilisera par exemple un filtre numérique passe-haut ayant une fréquence de coupure à la fréquence f1, en série avec un filtre numérique passe-bas ayant une fréquence de coupure à la fréquence f2. Il est également possible d'utiliser un filtre sélectif récursif à réponse impulsionnelle infinie (filtre RII) centré sur la bande de fréquence [f1 ;f2].

Le filtre passe haut (fréquence de coupure f1) a pour objectif de filtrer les basses fréquences inférieures à 0,1Hz, et incidemment de supprimer de nombreux artefacts dans le signal. En pratique la fréquence de coupure f1 est donc supérieure ou égale à 0,1 Hz, et de préférence comprise entre 0,1 Hz et 0,15Hz. Il permet également avantageusement de supprimer la valeur moyenne du signal. Il est envisageable de ne pas réaliser de filtre passe-haut. Dans ce cas, il est préférable, avant de calculer les paramètres intermédiaires (Aⱼ) de recentrer le signal sur sa moyenne.

Le filtre passe-bas (fréquence de coupure f2) a pour objectif de filtrer les hautes fréquences, typiquement supérieures à 1 Hz, car en pratique elles ne contiennent pas d'information intéressante. La mise en de ce filtrage passe-bas, bien que préférentielle, est toutefois facultative et non indispensable à la réalisation de l'invention.

### Etape 4 : Normalisation du signal

Cette étape est réalisée au moyen d'un sous-module logiciel qui dans un premier temps calcule la norme du signal issu de l'étape 3, c'est-à-dire la somme des carrés des valeurs du signal, divisée de préférence par le nombre d'échantillons N, et qui dans un deuxième temps effectue une normalisation du signal en divisant chaque valeur du signal par la norme précédemment calculée.

Cette étape 4 permet d'obtenir une meilleure sensibilité sur le résultat final (sensibilité des paramètres AUCmoy et AUCmax mesurant le niveau de douleur).

Il est essentiel que cette normalisation soit effectuée sur toute la largeur de la fenêtre principale, c'est-à-dire en prenant en compte dans la normalisation les (N) échantillons RRi de la fenêtre principale. Par contraste, une normalisation effectuée sur chaque sous-fenêtre (Aⱼ) serait totalement inefficace.

### Etape 5 : détection des minimums dans la fenêtre principale

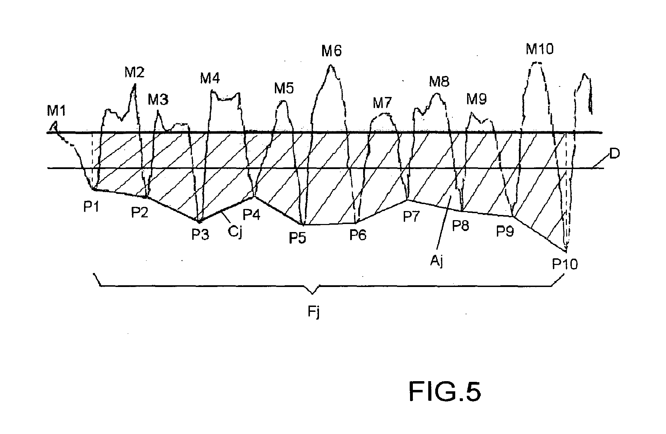

On a représenté sur la figure 5, un exemple de signal RR (après filtrage et normalisation) issu de l'étape 4 précitée. Sur cette figure, l'axe des abscisses correspond à la valeur nulle du signal filtré et normalisé.

L'étape 5 est réalisée au moyen d'un sous-module logiciel qui détecte les minimums (points Pᵢ sur la figure 5) de ce signal, par exemple au moyen d'un algorithme détectant une inversion de la pente (ou de manière équivalente un changement du signe de la dérivée du signal).

D'un point de vue physiologique, chaque minium correspond à un pic respiratoire.

### Etape 6 : Découpage de la fenêtre temporelle principale en m [m ≥2] sous-fenêtres (Fⱼ) et calcul pour chaque sous-fenêtre (Fⱼ) de l'aire (Aⱼ) délimitée par l'enveloppe inférieure (Cⱼ), reliant les minimums détectés à l'étape précédente.

Le sous-module logiciel correspondant à l'étape 6 calcule automatiquement, pour chaque sous-fenêtre (Fⱼ), une enveloppe inférieure (courbe Cⱼ sur la figure 5), qui relie les minimums (points Pᵢ) détectés à l'étape 5 et compris dans la sous-fenêtre (Fⱼ). Cette enveloppe inférieure (Cⱼ) est par exemple déterminée par régression linéaire, c'est-à-dire en calculant les segments de droite reliant les points minimums (Pi).

Ensuite le sous-module logiciel correspondant à l'étape 6 calcule, pour chaque sous-fenêtre (Fⱼ), l'aire (Aⱼ) délimitée par l'enveloppe inférieure (Cⱼ) et l'axe des abscisses (valeur nulle du signal) L'aire (Aⱼ) correspond à la surface hachurée sur la figure 5.

Lorsque l'enveloppe inférieure (Cⱼ) est approximée par des segments de droite, ce calcul d'aire (Aⱼ) revient à calculer la somme des aires des trapèzes délimités par deux point minimum (Pi) successifs et l'axe des abscisses.

Dans une autre variante de réalisation, l'aire (Aⱼ) pourrait être délimitée par l'enveloppe inférieure (Cⱼ) et une droite horizontale différente de l'axe des abscisses, et de préférence positionnée sous l'axe des abscisses, tel que par exemple la droite D en pointillés sur la figure 5.

A titre d'exemple non limitatif, le procédé a été testé avec une durée de fenêtre principale valant 64s, et pour la mise en oeuvre de l'étape 6, cette fenêtre principale était découpée en quatre (m=4) sous-fenêtres F₁ à F₄ de 16s.

### Etape 7 : Calcul des paramètres : AUCmoy et AUCmax

Les deux paramètres AUCmoy et AUCmax permettent de mesurer le niveau de douleur.

AUCmoy est égal (ou plus généralement proportionnel) à la moyenne des aires Aj des (m) sous-fenêtres Fⱼ.

AUCmax est égal (ou plus généralement proportionnel) à la valeur de la plus grande des aires Aj calculées pour les (m) sous-fenêtres Fⱼ.

### Etape 8 : Décalage, d'un pas temporel valant p secondes (p<n), de la fenêtre temporelle de n secondes, et réitération du calcul à partir de l'étape 2.

Le pas (p) pour le glissement de la fenêtre principal de calcul et réitération des étapes 2 à 8 influe sur la sensibilité des paramètres AUCmoy et AUCmax, et dépend donc de la sensibilité souhaitée. A titre indicatif, et en pratique, des résultats très satisfaisants ont été obtenus en choisissant un pas (p) valant 2s.

Dans une autre variante de réalisation, l'étape de filtrage précitée (Etape 3) pourrait être effectuée avant l'étape 2 de sélection, par exemple en continu au fur et à mesure de l'acquisition des échantillons RRi. Egalement, dans une autre variante de réalisation, cette étape de filtrage pourrait être réalisée après l'étape de normalisation (étape 4 précitée).

### Validation expérimentale

Des essais ont montré que le paramètre AUCmoy et le paramètre AUCmax étaient chacun corrélés à l'intensité du stimulus douloureux ressenti par un être vivant, ces paramètres permettant ainsi de mesurer l'intensité de la douleur chez un être vivant.

Afin de valider cette corrélation entre les paramètres précités AUCmoy et AUCmax et le niveau de douleur ressenti, ces paramètres AUCmoy et AUCmax ont été testés sur une population de parturientes majeures, sans antécédent cardio-vasculaire, diabétique ou neurologique, classées ASA I ou II, devant accoucher à terme et par voie basse sous analgésie péridurale (APD).

Les valeurs calculées AUCmoy et AUCmax ont été comparées avec les indications de niveau de douleur fournies par les parturientes au moyen d'une échelle visuelle analogique de la douleur (EVA), avant mise en place de la péridurale et après mise en place de la péridurale.

Cette comparaison a permis de démontrer que les paramètres AUCmoy et AUCmax étaient corrélés avec les indications de niveau de douleur fournies par les parturientes au moyen de l'EVA.

Il ressort plus particulièrement que le paramètre AUCmax est le paramètre le plus fiable, et est donc préférentiel.

Les deux paramètres AUCmax et AUCmoy ont été testés uniquement sur des patients conscients, et on a pu constater que de manière avantageuse selon l'invention, ces paramètres sont indépendants de la fréquence respiratoire propre de l'être vivant.

Néanmoins, on peut généraliser également l'utilisation de ces paramètres pour l'évaluation de la douleur chez un être vivant non conscient, et a fortiori pour un être vivant sous anesthésie générale dont la fréquence respiratoire est imposée par un mécanisme de ventilation contrôlée. La connaissance en anesthésie générale de ces paramètres permet indirectement de mesurer la composante analgésique de l'anesthésie générale.

L'invention n'est toutefois pas limitée aux deux paramètres particuliers précités AUCmax et AUCmoy, qui ont été donnés uniquement à titre d'exemples préférés.

Plus généralement, il a été constaté qu'un élément essentiel de l'invention influant sur le résultat visé (évaluation de la douleur) était le calcul d'un paramètre final à partir de paramètres intermédiaires calculés sur les échantillons RRi compris dans des sous-fenêtre temporelles, par contraste avec une solution (non couverte par l'invention) dans laquelle le paramètre final serait calculé directement sur l'ensemble des échantillons RRi de la fenêtre principale. Dans sa généralité, le procédé de l'invention pourra donc être mis en oeuvre avec toute méthode de traitement des échantillons RRi permettant de calculer un paramètre intermédiaire Aj dans des sous-fenêtres temporelles, le paramètre final corrélé avec l'intensité du stimulus douloureux, étant fonction de ces paramètres intermédiaires.

Egalement, il a été mis en évidence que de manière inattendue le stimulus douloureux influait très sensiblement sur les pics respiratoires (points minimum Pi) ; plus l'intensité de la douleur est importante, et plus l'amplitude des pics dans le signal correspondant aux minimums (Pi) est faible. En revanche, les maximums (Mi) de la courbe ne sont pas sensiblement affectés par le stimulus douloureux. En conséquence, tout méthode de traitement des échantillons RRi dans chaque sous-fenêtre (Fj) permettant de prendre en compte les variations d'amplitude de ces pics sera appropriée pour calculer les paramètre intermédiaires dans chaque sous-fenêtre, et l'invention n'est donc pas limitée au calcul d'aire précédemment décrit et donné à titre uniquement d'exemple. A titre d'exemple non exhaustifs, le calcul des aires (Aj) pourrait être remplacé par le calcul d'un paramètre intermédiaire fonction des amplitudes des pics correspondant aux points minimum (Pi). Egalement , dans une autre variante de réalisation, il est possible de calculer chaque paramètre intermédiaire (Aⱼ) en appliquant sur les échantillons RRi de la sous-fenêtre (Fⱼ) une transformée en ondelettes, basée sur une ondelette de forme appropriée (par exemple ondelette de Daubechies).

## Revendications

1. Procédé d'évaluation de la douleur ressentie par un être vivant, **caractérisé en ce qu'**il comprend les étapes principales suivantes :
- acquisition d'un signal cardiaque analogique de l'être vivant,
- échantillonnage de ce signal cardiaque et construction d'une série RR constituée d'une pluralité d'échantillons (RRᵢ) représentant les intervalles de temps qui séparent deux battements cardiaques successifs,
- sélection de (N) échantillons (RRi) dans une fenêtre temporelle principale de durée (n) prédéfinie,
- découpage de cette fenêtre principale en (m) sous-fenêtres (Fⱼ),
- calcul pour chaque sous-fenêtre (Fⱼ) d'un paramètre intermédiaire (Aⱼ) à partir des échantillons (RRi) compris dans la sous-fenêtre (Fⱼ),
- calcul d'un paramètre final qui est fonction des paramètres intermédiaires (Aⱼ).

2. Procédé selon la revendication 1 **caractérisé en ce qu'**on calcule de manière itérative le paramètre final en décalant la fenêtre temporelle principale d'un pas de durée (p) prédéfinie inférieure à la durée (n) de la fenêtre principale.

3. Procédé selon la revendication 1 ou 2 **caractérisé en ce que** chaque paramètre intermédiaire (Aⱼ) est fonction des minimums (Pi) de la série RR dans la sous-fenêtre (Fⱼ).

4. Procédé selon la revendication 3 **caractérisé en ce que** la valeur calculée pour le paramètre final (AUCmax) caractérisant l'intensité de la douleur est proportionnelle à la valeur maximale des paramètres intermédiaires (Aⱼ).

5. Procédé selon la revendication 3 **caractérisé en ce que** la valeur calculée pour le paramètre final (AUCmoy) caractérisant l'intensité de la douleur est proportionnelle à la moyenne des valeurs des paramètres intermédiaires (Aj).

6. Procédé selon l'une des revendications 1 à 5 **caractérisé en ce que** pour calculer les paramètres intermédiaires (Aⱼ), on détermine une enveloppe inférieure (Cⱼ) reliant les points minimums (Pi) détectés.

7. Procédé selon la revendication 6 **caractérisé en ce que** chaque paramètre intermédiaire (Aⱼ) est fonction d'une aire délimitée par l'enveloppe inférieure (Cⱼ).

8. Procédé selon l'une quelconque des revendications 1 à 7 **caractérisé en ce que** préalablement au calcul du paramètre, on filtre la série RR au moyen d'un filtre passe-haut présentant une fréquence de coupure f1 supérieure ou égale à 0,1Hz, et de préférence comprise entre 0,1Hz et 0,15Hz.

9. Procédé selon l'une quelconque des revendications 1 à 8 **caractérisé en ce que** préalablement au calcul des paramètres intermédiaires (Aⱼ) dans chaque sous fenêtre (Fⱼ), on normalise les échantillons (RRi) de la série RR sur toute la largeur de la fenêtre principale.

10. Dispositif pour l'évaluation de la douleur chez un être vivant **caractérisé en ce qu'**il comprend des moyens (1, 2) d'acquisition d'un signal cardiaque analogique, des moyens (4) d'échantillonnage de ce signal cardiaque, des moyens (5) de traitement du signal échantillonné, lesquels moyen de traitement (5) sont conçus pour calculer automatiquement au moins un paramètre conformément au procédé visé à l'une quelconque des revendication 1 à 9.
